(19) Europäisches Patentamt
European Patent Office
Office européen des brevets

(11) Veröffentlichungsnummer: **0 233 842 B1**

(12) **EUROPÄISCHE PATENTSCHRIFT**

(45) Veröffentlichungstag der Patentschrift: **27.05.92**

(51) Int. Cl.$^5$: **A61L 2/18**, G02C 13/00, A01N 37/44, A01N 57/12

(21) Anmeldenummer: **87810086.6**

(22) Anmeldetag: **13.02.87**

# (54) Lösungen amphoterer Tenside zur Reinigung oder Konservierung von Weichkontaktlinsen.

(30) Priorität: **19.02.86 US 831221**

(43) Veröffentlichungstag der Anmeldung:
**26.08.87 Patentblatt 87/35**

(45) Bekanntmachung des Hinweises auf die Patenterteilung:
**27.05.92 Patentblatt 92/22**

(84) Benannte Vertragsstaaten:
**AT BE CH DE ES FR GB GR IT LI LU NL SE**

(56) Entgegenhaltungen:
**US-A- 4 354 952**

(73) Patentinhaber: **CIBA-GEIGY AG**
**Klybeckstrasse 141**
**CH-4002 Basel(CH)**

(72) Erfinder: **Tsao, Fu-Pao**
**1361 Pinehurst Hunt**
**Lawrenceville Georgia 30245(US)**
Erfinder: **Penley, Charles Allen**
**691 Juniper**
**Atlanta Georgia 30308(US)**

**EP 0 233 842 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Lösungen zur Reinigung oder Konservierung einer Weichkontaktlinse, die ein Tensid enthalten, das gleichzeitig der Konservierung der Lösung dient.

Einige Lösungen zum Reinigen von Kontaktlinsen sind bereits bekannt. Ueblicherweise enthalten solche Lösungen entweder Sorbinsäure, Thimerosal, Chlorhexidin, ein polyquaternäres Germizid, ein synthetisches Antibiotikum oder ein übliches quaternäres Germizid, wie Benzalkoniumchlorid, als Konservierungsstoff. Diese herkömmlichen Konservierungsstoffe haben aber Nachteile, die ihre Verwendungsmöglichkeiten einschränken. So enthält beispielsweise Sorbinsäure üblicherweise Rückstände von Formaldehyd, Thimerosal kann bei manchen Patienten Allergien auslösen, Chlorhexidin ist verhältnismässig toxisch, und Benzalkoniumchlorid neigt dazu, sich im Laufe der Zeit im Material von hydrophilen Weichkontaktlinsen anzusammeln, an die Hornhaut abgegeben zu werden und so Beschwerden im Auge hervorzurufen.

In der US-PS 4,046,706 wird die Verwendung bestimmter amphoterer Tenside auf der Basis von Imidazol in Reinigungslösungen für Linsen offenbart. Solche Lösungen müssen normalerweise auch ein germizides Konservierungsmittel der vorstehend angesprochenen Art enthalten.

Ueberraschenderweise und unerwarteterweise wurde nun gefunden, dass die erfindungsgemäss zu verwendenden amphoteren Tenside, obwohl sie auch ein quartäres Stickstoffatom aufweisen, sich nicht nennenswert in herkömmlichen hydrophilen Weichkontaktlinsen anreichern. Dies ist ein gravierender Unterschied zu herkömmlichen quartären Germiziden, wie Benzalkoniumchloriden (dabei handelt es sich um durch Alkyl substituiertes Dimethylbenzylammoniumchlorid, worin Alkyl ein Gemisch von acht bis achtzehn Kohlenstoffatome enthaltenden Alkylgruppen bedeutet), Cetylpyridiniumchlorid und Dodecyltriethanolaminhydrochlorid, die dazu neigen, sich im Material von hydrophilen Weichkontaktlinsen anzureichern. Weiterhin neigen die erfindungsgemäss zu verwendenden amphoteren Tenside nicht dazu, sich im Auge anzureichern und sind üblicherweise weniger stark reizauslösend als die herkömmlichen quartären Germizide.

Verbindungen wie die erfindungsgemäss verwendeten amphoteren Tenside wurden von Riedhammer (US-A-4,354,952) bereits als Bestandteil von Lösungen offenbart, die für die Desinfektion und Konservierung von Kontaktlinsen vorgesehen sind. Diese Lösungen enthalten jedoch obligatorisch Chlorhexidin oder ein Salz davon. Zwar ist es gemäss Riedhammer möglich, mit verhältnismässig geringen Mengen Chlorhexidin auszukommen. Dies gilt jedoch nur, wenn spezielle, niedrig-dosierte Kombinationen von drei unterschiedlichen Bestandteilen als antimikrobielles Mittel verwendetwerden, nämlich Chlorhexidin, amphoteres Tensid und nicht-ionisches Tensid. Demgegenüber wird gemäss der vorliegenden Erfindung auf Konservierungsmittel wie Chlorhexidin verzichtet, da überraschenderweise gewisse amphotere Tenside allein als Konservierungsmittel geeignet sind.

Ein Gegenstand der vorliegenden Erfindung besteht daher darin, die verschiedenartigen Nachteile auszuräumen, die mit der Verwendung der bisher bekannten Reinigungslösungen für Weichkontaktlinsen verbunden sind.

Ein weiterer Gegenstand der vorliegenden Erfindung besteht darin, ein Verfahren zur Reinigung und Konservierung von hydrophilen Weichkontaktlinsen zur Verfügung zu stellen, wobei im Hinblick auf die gewünschten Eigenschaften wirksame Mengen der erfindungsgemässen amphoteren Tenside verwendet werden.

Ein weiterer Gegenstand der vorliegenden Erfindung besteht darin, Zusammensetzungen zur Verfügung zu stellen, die in solchen Verfahren verwendet werden können sowie Verfahren zu deren Herstellung.

So bezieht sich die vorliegende Erfindung auf wässrige, sich selbst konservierende Reinigungs- oder Konservierungslösungen für Weichkontaktlinsen, die als einziges Konservierungsmittel eine im Hinblick auf die gewünschten Eigenschaften als Tensid und Konservierungsmittel wirksame Menge eines wasserlöslichen amphoteren Tensids der Formel I enthalten,

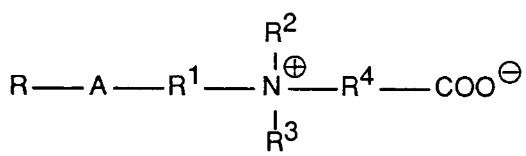

(I)

worin R für Alkyl, Alkenyl oder Alkandienyl mit 6 bis 18 Kohlenstoffatomen steht, jeder dieser Reste unsubstituiert oder durch Halogen, Hydroxy oder Amino substituiert, A für -O-, -S-, -C(O)O- oder-C(O)NR'- steht, worin R' Wasserstoff oder Niederalkyl bedeutet, $R^1$ für unsubstituiertes oder durch Hydroxy substituiertes Alkylen mit 2 bis 6 Kohlenstoffatomen steht; $R^2$ und $R^3$ unabhängig voneinander Wasserstoff oder Niederalkyl bedeuten, das unsubstituiert oder durch Carboxy oder durch ein oder zwei Hydroxygruppen

substituiert ist, von denen eine mit Phosphor- oder Schwefelsäure verestert sein kann, und $R^4$ unsubstituiertes oder durch Hydroxy substituiertes Alkylen mit bis zu 3 Kohlenstoffatomen bedeutet, oder ein ophthalmologisch annehmbares Salz davon,
sowie ein ophthalmologisch annehmbares wasserlösliches, mit dem Augengewebe verträgliches Salz in ausreichender Menge, so dass die Lösung einen Salzgehalt erhält, der einer Tonizität von etwa 0,5 bis etwa 2 Gew.-% Natriumchlorid entspricht.

Für den Fall, dass A für -C(O)O- oder-C(O)NR'- steht, ist die Carbonylgruppe dieser bivalenten Reste vorzugsweise an den Rest R gebunden.

Alkyl mit 6 bis 18 Kohlenstoffatomen hat vorzugsweise 8 bis 18 Kohlenstoffatome und ist z.B. Octyl, Decyl, Undecyl, Dodecyl, Tridecyl, Pentadecyl, Hexadecyl, Heptadecyl, Octadecyl oder ein Gemisch davon.

Alkenyl mit 6 bis 18 Kohlenstoffatomen hat vorzugsweise 8 bis 18 Kohlenstoffatome und ist z.B. Octenyl, Decenyl, Undecenyl oder Heptadecenyl, wie Heptadec-8-enyl.

So wird für den Fall, dass A für-C(O)NH- steht, die Teilstruktur R-A- in Verbindungen der Formel I z.B. durch $C_{11}$-$C_{17}$-Alkylcarbonylamino oder $C_{17}$-Alkenylcarbonylamino repräsentiert, wie Lauroylamino, Myristoylamino, Palmitoylamino, Stearoylamino, Oleoylamino, oder ein Gemisch davon, wie Cocoylamino. Letzteres wird auch Cocoamido genannt, wird aus natürlichen Rohstoffen gewonnen und hat im Durchschnitt 12 Kohlenstoffatome.

Halogen ist z.B. Fluor, Iod oder vorzugsweise Chlor und Brom.

Niederalkyl hat z.B. bis zu 7 Kohlenstoffatome, vorzugsweise bis zu 4 Kohlenstoffatome, und ist z.B. Methyl, Ethyl, Propyl, Butyl oder tert.-Butyl.

Alkylen mit 2 bis 6 Kohlenstoffatomen ist z.B. 1,2-Ethylen, 1,3-Propylen oder 1,4-Butylen. Diese Alkylengruppen können durch Hydroxy substituiert sein, so dass sich z.B. Hydroxypropylen oder Hydroxybutylen ergibt, vorzugsweise 2-Hydroxy-1,3-propylen.

Vorzugsweise ist A -C(O)NH- und $R^1$ ist Alkylen mit 2 bis 3 Kohlenstoffatomen.

$R^2$ und $R^3$ sind vorzugsweise unabhängig voneinander unsubstituiertes oder durch Hydroxy mono- oder disubstituiertes Niederalkyl, wobei jeweils eine Hydroxygruppe davon unter Bildung des entsprechenden Mono-, Di- oder Tri-Organophosphorsäureesters mit Phosphorsäure verestert sein kann.

$R^4$ ist vorzugsweise unsubstituiertes Alkylen mit bis zu 3 Kohlenstoffatomen und bedeutet insbesondere Methylen, 1,2-Ethylen, 1,1-Ethylen, 1,3-Propylen oder 1,2-Propylen. In einer anderen bevorzugten Ausführungsform bedeutet $R^4$ 2-Hydroxy-1,3-propylen.

Bevorzugt sind Lösungen, die als einziges Konservierungsmittel ein Tensid der Formel I enthalten, worin R Alkyl oder Alkenyl mit 11 bis 17 Kohlenstoffatomen ist, A für -O- oder -C(O)NH- steht, dessen Carbonylgruppe an R gebunden ist, $R^1$ für Alkylen mit 2 oder 3 Kohlenstoffatomen steht, welches unsubstituiert oder durch Hydroxy substituiert ist, $R^2$ und $R^3$ unabhängig voneinander für unsubstituiertes oder durch Carboxy monosubstituiertes oder durch Hydroxy mono- oder disubstituiertes Niederalkyl stehen, wobei eine dieser Hydroxygruppen unter Bildung des entsprechenden Triorganophosphats mit Phosphorsäure verestert sein kann, und $R^4$ Alkylen mit bis zu 3 Kohlenstoffatomen ist, oder ophthalmologisch annehmbare Salze davon.

Besonders bevorzugt sind Lösungen, die als einziges Konservierungsmittel ein Tensid der Formel I enthalten, worin R Alkyl mit 11 bis 17 Kohlenstoffatomen oder Alkenyl mit 17 Kohlenstoffatomen ist, A für -C(O)NH- steht, dessen Carbonylgruppe an R gebunden ist, $R^1$ für Alkylen mit 2 oder 3 Kohlenstoffatomen steht, $R^2$ und $R^3$ unabhängig voneinander für unsubstituiertes oder durch Hydroxy monosubstituiertes Niederalkyl stehen, wobei diese Hydroxygruppe unter Bildung des entsprechenden Triorganophosphats mit Phosphorsäure verestert sein kann, und $R^4$ Alkylen mit bis zu 3 Kohlenstoffatomen ist, oder ophthalmologisch annehmbare Salze davon.

Eine andere bevorzugte Gruppen umfasst Lösungen, die als einziges Konservierungsmittel ein Tensid der Formel I enthalten, worin R Alkyl oder Alkenyl mit 8 bis 12 Kohlenstoffatomen ist, A für -C(O)NH- steht, dessen Carbonylgruppe an R gebunden ist, $R^1$ für Alkylen mit 2 oder 3 Kohlenstoffatomen steht, $R^2$ und $R^3$ unabhängig voneinander für Niederalkyl stehen, $R^4$ Alkylen mit bis zu 3 Kohlenstoffatomen ist, oder ophthalmologisch annehmbare Salze davon.

Bevorzugte Verbindungen der Formel I sind beispielsweise:
N-Lauroylaminopropyl-N,N-dimethylglycin;
N-Cocoylaminopropyl-N,N-dimethylglycin;
N-Lauroylaminopropyl-N-carboxymethyl-N-hydroxyethylglycin;
N-Oleoylaminopropyl-N-carboxymethyl-N-hydroxyethylglycin;
N-3-Dodecyloxy-2-hydroxypropyl-N,N-dimethylglycin;
N-Cocoylaminopropyl-N-hydroxyethyl-3-aminopropionsäure, und
Tri-[3-(N-cocoylaminoethyl-N-hydroxyethyl-N-carboxymethyl)amino-2-hydroxy-propanol]phosphat.

Besonders bevorzugt sind N-Lauroylaminopropyl-N,N-dimethylglycin; N-Cocoylaminopropyl-N,N-dimethylglycin und Tri-[3-(N-cocoylaminoethyl-N-hydroxyethyl-N-carboxymethyl)amino-2-hydroxy-propanol]-phosphat.

Geeignete ophthalmologisch annehmbare Salze lassen sich aus den Verbindungen der Formel I z.B. mit Natriumchlorid bilden, ferner mit Kaliumchlorid, Natriumphosphat, Natriumborat, Natriumbicarbonat, Kohlensäure, Borsäure, Diethanolamin und analogen anorganischen und organischen Säuren, Basen und Salzen, die üblicherweise in Lösungen zur Linsenpflege angewendet werden. Wie bekannt kann, wenn Salze wie Natriumchlorid verwendet werden, die zwitterionische innere Salzform im Gleichgewicht mit der entsprechenden Doppelsalzform stehen.

Wenn vor- oder nachstehend davon die Rede ist, dass die erfindungsgemässen Lösungen eine Verbindung der Formel I oder ein Salz davon enthalten, so ist damit stets auch gemeint, dass eine solche Lösung ein Gemisch von zwei oder mehr Verbindungen der Formel I enthalten kann, von zwei oder mehr Salzen davon oder ein Gemisch von einer oder mehreren Verbindungen der Formel I mit einem oder mehreren Salzen davon.

Eine erste Ausführungsform dieser Erfindung sind wässrige, sich selbst konservierende Reinigungslösungen für Weichkontaktlinsen. Diese können zusätzlich zur Verbindung der Formel I andere übliche Zusätze und Hilfsmittel enthalten. Beispielsweise enthalten geeignete sich selbst konservierende Reinigungslösungen bezogen auf das Gesamtgewicht der Lösung:

a) zwischen etwa 0,005 und etwa 2,0 Gew.-% einer Verbindung der Formel I,
b) zwischen 0 und etwa 5 Gew.-% eines vorwiegend nichtionischen Tensids,
c) zwischen 0 und etwa 5 Gew.-% eines Verdickungsmittels,
d) zwischen 0 und etwa 1 Gew.-% eines Chelatbildners,
e) zwischen 0 und etwa 2 Gew.-% eines Puffers,
f) zwischen 0,5 und etwa 2 Gew.-% eines wasserlöslichen Salzes, das mit dem Augengewebe verträglich ist, und
g) im übrigen Wasser.

Vorzugsweise enthalten die erfindungsgemässen Zusammensetzungen zwischen etwa 0,01 und etwa 0,5 Gew.-%, besonders bevorzugt zwischen etwa 0,02 und etwa 0,2 Gew.-% einer Verbindung der Formel I.

Falls sie anwesend sind, sind die Komponenten b)bis f) vorteilhafterweise jeweils in einer Mindestmenge von etwa 0,01 Gew.-% anwesend, bezogen auf das Gesamtgewicht der Lösung.

Geeignete vorwiegend nichtionische Tenside zur Verwendung als Komponente b) umfassen vorzugsweise diejenigen nichtionischen Tenside, von denen bekannt ist, dass sie allgemein mit Augengewebe verträglich sind, und umfassen physiologisch annehmbare Poly(oxyethylen)-poly(oxypropylen)-Blockcopolymere, wie die unter dem Namen Pluronic® von der Wyandotte Chemical Corporation vertriebenen Copolymere, die sich durch die folgende empirische Formel wiedergeben lassen:

$$HO(CH_2CH_2O)_a(CH(CH_3)CH_2O)_b(CH_2CH_2O)_cH$$

Hierin sind a und c statistisch gleich, das durchschnittliche Molekulargewicht bewegt sich zwischen 2000 und 16000, und die Polyoxyethyleneinheiten machen zwischen etwa 10 und 80 Gew.-% des Moleküls aus.

Ebenfalls geeignet sind beispielsweise polyethoxylierte Alkylphenole, deren Alkylteile etwa zwischen 6 und 12 Kohlenstoffatome enthalten und die zwischen etwa 3 und etwa 50 Polyoxyethyleneinheiten je Molekül enthalten, wie auch die entsprechenden alkylierten Methylenbisphenole, die polyethoxyliert sind, wie dasjenige, das von der Ruger Chemical Company unter der Bezeichnung Tyloxapol® vertrieben wird.

Ebenfalls geeignet sind polyethoxylierte Fettalkohole, Fettsäuren und Fettamine, z.B. solche, deren Alkyleinheiten zwischen 6 und 18 Kohlenstoffatome enthalten und die im Mittel zwischen etwa 3 und etwa 50 Polyoxyethyleneinheiten aufweisen. Vorzugsweise enthalten die Zusammensetzungen zwischen 0 und etwa 0,5 Gew.-% der Komponente b).

Geeignete Verdickungsmittel c) umfassen diejenigen, die herkömmlicherweise in ophthalmischen Formulierungen verwendet werden, beispielsweise Hydroxyethylcellulose, Hydroxypropylcellulose, Polyvinylalkohol, Polyvinylpyrrolidon, Polyethylenglykol und dergleichen. Vorzugsweise ist ein Verdickungsmittel in Mengen zwischen 0 und etwa 2 Gew.-% anwesend, bezonders bevorzugt in Mengen zwischen 0 und etwa 1,5 Gew.-%.

Geeignete Chelatbildner d) umfassen herkömmliche Chelatbildner wie Ethylendiamintetraessigsäure (EDTA) und Alkalisalze, wie die Di-oder Trinatriumsalze davon, und Trihydroxymethylaminomethan. Vorzugsweise ist der Chelatbildner in einer Menge zwischen 0 und etwa 0,7 Gew.-% anwesend.

Geeignete Puffer e) umfassen Alkalimetallphosphate, -citrate, -borate, -tartrate, -acetate, -carbonate,

-bicarbonate und die entsprechenden Gemische davon mit Säuren, wie Phosphorsäure, Zitronensäure, Borsäure, Weinsäure, und Gemische davon. Vorzugsweise sind Puffer in Mengen zwischen 0 und etwa 1 Gew.-% anwesend.

Geeignete wasserlösliche Salze f), die mit dem Augengewebe verträglich sind, umfassen insbesondere diejenigen Salze, die herkömmlicherweise verwendet werden, um Lösungen herzustellen, die einen Salzehalt haben, der einem Gehalt von bis zu etwa 0,9 % Natriumchlorid entspricht. Bevorzugte Salze sind Alkalimetallhalogenide, -sulfate, -nitrate und -phosphate, insbesondere Natriumchlorid, Kaliumchlorid und Gemische davon.

Eine andere Ausführungsform der Erfindung besteht in wässrigen, sich selbst konservierenden Konservierungslösungen für Weichkontaktlinsen. Auch diese enthalten eine die Lösung konservierende Menge der Verbindung der Formel I und eine ausreichende Menge eines wasserlöslichen, mit dem Augengewebe verträglichen Salzes, so dass die Lösung einen Salzgehalt erhält, der einer Tonizität von etwa 0,5 bis etwa 2 Gew.-% Natriumchlorid entspricht.

In dieser Ausführungsform ist es bevorzugt, einen Salzgehalt zu haben, der der Lösung einen Salzgehalt verleiht, der einer Tonizität zwischen etwa 0,7 und etwa 1,8 Gew.-% und besonders bevorzugt zwischen etwa 0,7 und etwa 1,0 Gew.-% Natriumchlorid entspricht.

Geeignete Salze, die zur Einstellung der Tonizität verwendet werden können, umfassen Alkalimetallhalogenide, -sulfate, -nitrate, -phosphate und dergleichen, insbesondere die Natrium- und Kaliumsalze davon und ganz besonders bevorzugt Natriumchlorid, Kaliumchlorid und Gemische davon.

Die Lösungen zur Konservierung und Aufbewahrung von Linsen können grundsätzlich die gleichen Zusätze und Hilfsmittel in den gleichen Mengen enthalten, die vorstehend im Zusammenhang mit den sich selbst konservierenden Reinigungslösungen für Kontaktlinsen genannt wurden, mit der Ausnahme, dass die Lösungen zur Konservierung und Aufbewahrung vorteilhafterweise eine ausreichende Menge der Komponente f) enthalten, um einen Salzgehalt zu erzielen, der einer Tonizität zwischen etwa 0,7 und etwa 1,8 Gew.-% Natriumchlorid entspricht.

Mit den erfindungsgemässen Lösungen können herkömmliche hydrophile Weichkontaktlinsen von Proteinen, Lipiden und anderen nicht proteinhaltigen Ablagerungen gereinigt werden, die sich normalerweise auf solchen Linsen festsetzen. Wenn diese Ablagerungen, die aus Schleim, Oelen, Kosmetika, Proteinen aus der Tränenflüssigkeit und dergleichen stammen, nicht entfernt werden, sammeln sie sich auf den Linsen an, verringern deren Lebensdauer und verschlechtern ihre optischen Eigenschaften. Die vorliegenden Zusammensetzungen sind ausserordentlich nützlich bei der Entfernung und Auflösung solcher Ablagerungen und daneben auch physiologisch annehmbar und selbstkonservierend.

Die bevorzugten erfindungsgemässen Lösungen zur Aufbewahrung und Konservierung weisen einen weiteren Vorteil auf. Er besteht darin, dass die Tonizität solcher Zusammensetzungen sowie der Umstand, dass die Verbindungen der Formel I das Auge nicht reizen, sowie ihre verminderte Neigung, sich in den Materialien von hydrophilen Weichkontaktlinsen anzusammeln, die Zusammensetzungen als äusserst geeignetes Medium zum Konsewieren von Linsen ausweisen. Aus einem solchen Medium kann eine Linse nämlich direkt in das Auge eingesetzt werden, ohne dass sie mit einer herkömmlichen Kochsalzlösung gespült werden müsste.

Die Zusammensetzungen der vorliegenden Erfindung werden aus Stoffen hergestellt, die als solche bekannt sind. So gehören die Verbindungen der Formel I zu einer bekannten Klasse von amphoteren Tensiden. Die Zusammensetzungen können auf herkömmliche Weise durch Vermischen der Bestandteile hergestellt werden.

Ein weiterer Gegenstand der Erfindung ist es, ein Verfahren zur Reinigung von Weichkontaktlinsen zur Verfügung zu stellen, das dadurch gekennzeichnet ist, dass eine Weichkontaktlinse intensiv mit einer wässrigen Lösung in Kontakt gebracht wird, die eine im Hinblick auf die gewünschten Eigenschaften als Tensid und Konservierungsmittel wirksame Menge eines wasserlöslichen Tensids der Formel I enthält. Ein geeignetes Verfahren zur Reinigung von Kontaktlinsen gemäss der Erfindung besteht z.B. darin, dass man die Linse auf eine Handfläche legt, einige Tropfen der Lösung auf beide Flächen der Linse gibt, die Oberflächen der Linse mit dem Zeigefinger oder zwischen Daumen und Zeigefinger reibt und mit weiterer Lösung spült.

Ferner ist es ein Gegenstand der Erfindung, ein Verfahren zur Konservierung von Weichkontaktlinsen zur Verfügung zu stellen, das dadurch gekennzeichnet ist, dass eine Kontaktlinse in einer Lösung aufbewahrt wird, die eine die Lösung konservierende Menge der Verbindung der Formel I und eine ausreichende Menge eines wasserlöslichen, mit dem Augengewebe verträglichen Salzes enthält, so dass die Lösung einen Salzgehalt erhält, der einer Tonizität von etwa 0,5 bis etwa 2 Gew.-% Natriumchlorid entspricht.

Die folgenden Beispiele dienen ausschliesslich der Illustrierung und sind nicht geeignet, den Umfang

der Erfindung zu beschränken. Alle Angaben von Prozentsätzen und Teilen beziehen sich auf das Gewicht falls nicht anderweitig angegeben.

Beispiel 1: Zu 0,5 ml einer wässrigen Lösung enthaltend 30 Gew.-% N-Cocoylaminopropyl-N,N-dimethylglycin (monateric® CAB von Mona Industries Inc.) werden 2,613 g Natriumchlorid, 1,25 g Natriumtetraborat, 1,75 g Borsäure, 1,0 ml Tyloxapol, 3,5 g Hydroxyethylcellulose und 2,5 g Dinatrium-EDTA gegeben. Unter Rühren wird mit Wasser auf ein Volumen von 500 ml aufgefüllt. Die erhaltene Lösung wird durch Ultrafiltration sterilfiltriert, man erhält so eine Lösung mit einem pH-Wert von 7,2 und einer Osmolarität von 304.

Beispiel 2: Zu 0,5 ml einer wässrigen Lösung enthaltend 30 Gew.-% N-Lauroylaminopropyl-N,N-dimethylglycin (Monateric® LMAB) werden 2,685 g Natriumchlorid, 1,25 g Tyloxapol, 3,5 g Hydroxyethylcellulose und 2,5 g Dinatrium-EDTA gegeben. Unter Rühren wird mit Wasser auf ein Volumen von 500 ml aufgefüllt. Die erhaltene Lösung wird durch Ultrafiltration sterilfiltriert, man erhält so eine Lösung mit einem pH-Wert von 7,2 und einer Osmolarität von 298.

Beispiel 3: Zu 0,5 ml einer wässrigen Lösung enthaltend 30 Gew.-% N-Cocoylaminopropyl-N,N-dimethylglycin werden 3,45 g Natriumchlorid, 0,7 g Natriumtetraborat, 2,7 g Borsäure und 1,0 g Dinatrium-EDTA gegeben. Unter Rühren wird mit Wasser auf ein Volumen von 500 ml aufgefüllt. Die erhaltene Lösung wird durch Ultrafiltration sterilfiltriert, man erhält so eine Lösung mit einem pH-Wert von 7,2 und einer Osmolarität von 306.

Beispiel 4: Zu 0,5 ml einer wässrigen Lösung enthaltend 30 Gew.-% N-Lauroylaminopropyl-N,N-dimethylglycin werden 3,18 g Natriumchlorid, 0,7 g Natriumtetraborat, 2,7 g Borsäure und 1,0 g Dinatrium-EDTA gegeben. Unter Rühren wird mit Wasser auf ein Volumen von 500 ml aufgefüllt. Die erhaltene Lösung wird durch Ultrafiltration sterilfiltriert, man erhält so eine Lösung mit einem pH-Wert von 7,21 und einer Osmolarität von 310.

Beispiel 5: Zu 0,3 g einer wässrigen Lösung enthaltend 30 Gew.-% Tris-[3-(N-cocoylaminoethyl-N-hydroxyethyl-N-carboxymethyl)amino-2-hydroxypropanol]phosphat (Monaquat P-TL von Mona Industries, Inc.) werden 0,24 g Tris(hydroxymethyl)aminomethan, 1,0 g Borsäure und 0,36 g Natriumchlorid gegeben, ferner soviel wasser, dass eine Lösung vom Volumen 100 ml erhalten wird.

Beispiel 6: Zu 1,5 g einer wässrigen Lösung enthaltend 30 Gew.-% N-Lauroylaminopropyl-N,N-dimethylglycin werden 2,685 g Natriumchlorid, 1,25 g Natriumtetraborat 1,75 g Borsäure 1,0 g Tyloxapol, 3,5 g Hydroxyethylcellulose und 2,5 g Dinatrium-EDTA gegeben, ferner unter Rühren soviel Wasser, dass ein Lösung mit einem Volumen von 500 ml erhalten wird. Der pH-Wert der Lösung beträgt 7,43. Er wird mit 0,5 n Salzsäure auf 7,23 eingestellt.

Beispiel 7: Die Vorgehensweise von Beispiel 6 wird wiederholt, indem gleiche Mengen von den Bestandteilen verwendet werden, ausser dass man von 2,5 g einer wässrigen Lösung enthaltend 30 Gew.-% N-Lauroylaminopropyl-N,N-dimethylglycin ausgeht. Nach Verdünnen mit Wasser auf 500 ml ergibt sich ein pH-Wert von 7,4, der mit 0,5 n Salzsäure auf 7,20 eingestellt wird.

Beispiel 8: Zu 1,5 g einer wässrigen Lösung enthaltend 30 Gew.-% N-Lauroylaminopropyl-N,N-dimethylglycin werden 1 g Dinatrium-EDTA, 2,7 g Borsäure, 0,70 g Natriumborat und 3,15 g Natriumchlorid gegeben. Unter Rühren wird soviel Wasser zugesetzt, dass eine Lösung vom Volumen 500 ml erhalten wird. Die Lösung weist einen pH-Wert von 7,22 auf und eine Osmolarität von 319.

Beispiel 9: Die Zusammensetzungen der Beispiele 6, 7 und 8 werden gemäss dem modifizierten Draize-Kaninchen-Test am Auge (Food, Drug and Cosmetic Law Reports 233) im Hinblick auf mögliche Reizungen überprüft. Jede der drei Lösungen erweist sich in diesem Test als "nicht-reizend".

## Patentansprüche

**Patentansprüch für folgende Vertragsstaaten : BE, CH, DE, FR, GB, GR, IT, LI, LU, NL, SE**

1. Wässrige, sich selbst konservierende Lösung, geeignet zur Reinigung oder Konservierung von Weichkontaktlinsen, enthaltend
   als einziges Konservierungsmittel eine im Hinblick auf die gewünschten Eigenschaften als Tensid und Konservierungsmittel wirksame Menge eines wasserlöslichen amphoteren Tensids der Formel I,

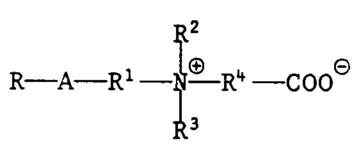

worin R für Alkyl, Alkenyl oder Alkandienyl mit 6 bis 18 Kohlenstoffatomen steht, jeder dieser Reste unsubstituiert oder durch Halogen, Hydroxy oder Amino substituiert, A für -O-, -S-, -C(O)O-oder -C(O)-NR'-steht, worin R' Wasserstoff oder Niederalkyl bedeutet, $R^1$ für unsubstituiertes oder durch Hydroxy substituiertes Alkylen mit 2 bis 6 Kohlenstoffatomen steht, $R^2$ und $R^3$ unabhängig voneinander Wasserstoff oder Niederalkyl bedeuten, das unsubstituiert oder durch Carboxy oder durch ein oder zwei Hydroxygruppen substituiert ist, von denen eine mit Phosphor- oder Schwefelsäure verestert sein kann, und $R^4$ unsubstituiertes oder durch Hydroxy substituiertes Alkylen mit bis zu 3 Kohlenstoffatomen bedeutet, oder ein ophthalmologisch annehmbares Salz davon,
sowie ein ophthalmologisch annehmbares wasserlösliches, mit dem Augengewebe verträgliches Salz in ausreichender Menge, so dass die Lösung einen Salzgehalt erhält, der einer Tonizität von etwa 0,5 bis etwa 2 Gew.-% Natriumchlorid entspricht.

**2.** Eine Lösung gemäss Anspruch 1, worin R Alkyl oder Alkenyl mit 11 bis 17 Kohlenstoffatomen ist, A für -0- oder -C(O)NH- steht, dessen Carbonylgruppe an R gebunden ist, $R^1$ für Alkylen mit 2 oder 3 Kohlenstoffatomen steht, welches unsubstituiert oder durch Hydroxy substituiert ist, $R^2$ und $R^3$ unabhängig voneinander für unsubstituiertes oder durch Carboxy monosubstituiertes oder durch Hydroxy mono- oder disubstituiertes Niederalkyl stehen, wobei eine dieser Hydroxygruppen unter Bildung des entsprechenden Triorganophosphats mit Phosphorsäure verestert sein kann, und $R^4$ Alkylen mit bis zu 3 Kohlenstoffatomen ist.

**3.** Eine Lösung gemäss Anspruch 1, worin R Alkyl mit 11 bis 17 Kohlenstoffatomen oder Alkenyl mit 17 Kohlenstoffatomen ist, A für -C(O)NH- steht, dessen Carbonylgruppe an R gebunden ist, $R^1$ für Alkylen mit 2 oder 3 Kohlenstoffatomen steht, $R^2$ und $R^3$ unabhängig voneinander für unsubstituiertes oder durch Hydroxy monosubstituiertes Niederalkyl stehen, wobei diese Hydroxygruppe unter Bildung des entsprechenden Triorganophosphats mit Phosphorsäure verestert sein kann, und $R^4$ Alkylen mit bis zu 3 Kohlenstoffatomen ist.

**4.** Eine Lösung gemäss Anspruch 1, worin R Alkyl oder Alkenyl mit 8 bis 12 Kohlenstoffatomen ist, A für -C(O)NH- steht, dessen Carbonylgruppe an R gebunden ist, $R^1$ für Alkylen mit 2 oder 3 Kohlenstoffatomen steht, $R^2$ und $R^3$ unabhängig voneinander für Niederalkyl stehen, $R^4$ Alkylen mit bis zu 3 Kohlenstoffatomen ist.

**5.** Eine Lösung gemäss Anspruch 1, worin die Verbindung der Formel I ausgewählt ist aus der Gruppe bestehend aus N-Lauroylaminopropyl-N,N-dimethylglycin, N-Cocoylaminopropyl-N,N-dimethylglycin und Tris-[3-(N-cocoylaminoethyl-N-hydroxyethyl-N-carboxymethyl)amino-2-hydroxypropanol]-phosphat.

**6.** Die Lösung gemäss Anspruch 1, die
a) zwischen etwa 0,005 und etwa 2,0 Gew.-% einer Verbindung der Formel I,
b) zwischen 0 und etwa 5 Gew.-% eines vorwiegend nichtionischen Tensids,
c) zwischen 0 und etwa 5 Gew.-% eines Verdickungsmittels,
d) zwischen 0 und etwa 1 Gew.-% eines Chelatbildners,
e) zwischen 0 und etwa 2 Gew.-% eines Puffers,
f) zwischen 0,5 und etwa 2 Gew.-% eines wasserlöslichen Salzes, das mit dem Augengewebe verträglich ist, und
g) im übrigen Wasser enthält.

**7.** Die Lösung gemäss Anspruch 6, die einen Salzgehalt hat, der einem Gehalt von bis zu etwa 0,9 % Natriumchlorid entspricht.

**8.** Verwendung einer Lösung gemäss Anspruch 6 zur Konservierung von Weichkontaktlinsen.

**9.** Verwendung einer Lösung gemäss Anspruch 7 zur Reinigung von Weichkontaktlinsen.

**10.** Verfahren zur Reinigung oder Konservierung einer Weichkontaktlinse, dadurch gekennzeichnet, dass eine solche Kontaktlinse intensiv mit einer Lösung gemäss Anspruch 1 in Kontakt gebracht wird.

**11.** Verfahren zur Herstellung einer Lösung gemäss Anspruch 1 durch herkömmliches Vermischen der Bestandteile.

**Patentansprüche für folgende Vertragsstaaten : AT, ES**

1. Verfahren zur Herstellung einer wässrigen, sich selbst konservierenden Lösung, geeignet zur Reinigung oder Konservierung von Weichkontaktlinsen, enthaltend
als einziges Konservierungsmittel eine im Hinblick auf die gewünschten Eigenschaften als Tensid und Konservierungsmittel wirksame Menge eines wasserlöslichen amphoteren Tensids der Formel I,

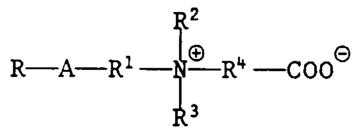

worin R für Alkyl, Alkenyl oder Alkandienyl mit 6 bis 18 Kohlenstoffatomen steht, jeder dieser Reste unsubstituiert oder durch Halogen, Hydroxy oder Amino substituiert, A für -O-, -S-, -C(O)O-oder -C(O)-NR'-steht, worin R' Wasserstoff oder Niederalkyl bedeutet, $R^1$ für unsubstituiertes oder durch Hydroxy substituiertes Alkylen mit 2 bis 6 Kohlenstoffatomen steht, $R^2$ und $R^3$ unabhängig voneinander Wasserstoff oder Niederalkyl bedeuten, das unsubstituiert oder durch Carboxy oder durch ein oder zwei Hydroxygruppen substituiert ist, von denen eine mit Phosphor- oder Schwefelsäure verestert sein kann, und $R^4$ unsubstituiertes oder durch Hydroxy substituiertes Alkylen mit bis zu 3 Kohlenstoffatomen bedeutet, oder ein ophthalmologisch annehmbares Salz davon,
sowie ein ophthalmologisch annehmbares wasserlösliches, mit dem Augengewebe verträgliches Salz in ausreichender Menge, so dass die Lösung einen Salzgehalt erhält, der einer Tonizität von etwa 0,5 bis etwa 2 Gew.-% Natriumchlorid entspricht, gekennzeichnet durch herkömmliches Vermischen der Bestandteile.

2. Verfahren gemäss Anspruch 1, worin R Alkyl oder Alkenyl mit 11 bis 17 Kohlenstoffatomen ist, A für -0- oder -C(O)NH- steht, dessen Carbonylgruppe an R gebunden ist, $R^1$ für Alkylen mit 2 oder 3 Kohlenstoffatomen steht, welches unsubstituiert oder durch Hydroxy substituiert ist, $R^2$ und $R^3$ unabhängig voneinander für unsubstituiertes oder durch Carboxy monosubstituiertes oder durch Hydroxy mono- oder disubstituiertes Niederalkyl stehen, wobei eine dieser Hydroxygruppen unter Bildung des entsprechenden Triorganophosphats mit Phosphorsäure verestert sein kann, und $R^4$ Alkylen mit bis zu 3 Kohlenstoffatomen ist.

3. Verfahren gemäss Anspruch 1, worin R Alkyl mit 11 bis 17 Kohlenstoffatomen oder Alkenyl mit 17 Kohlenstoffatomen ist, A für -C(O)NH-steht, dessen Carbonylgruppe an R gebunden ist, $R^1$ für Alkylen mit 2 oder 3 Kohlenstoffatomen steht, $R^2$ und $R^3$ unabhängig voneinander für unsubstituiertes oder durch Hydroxy monosubstituiertes Niederalkyl stehen, wobei diese Hydroxygruppe unter Bildung des entsprechenden Triorganophosphats mit Phosphorsäure verestert sein kann, und $R^4$ Alkylen mit bis zu 3 Kohlenstoffatomen ist.

4. Verfahren gemäss Anspruch 1, worin R Alkyl oder Alkenyl mit 8 bis 12 Kohlenstoffatomen ist, A für -C-(O)NH- steht, dessen Carbonylgruppe an R gebunden ist, $R^1$ für Alkylen mit 2 oder 3 Kohlenstoffatomen steht, $R^2$ und $R^3$ unabhängig voneinander für Niederalkyl stehen, $R^4$ Alkylen mit bis zu 3 Kohlenstoffatomen ist.

5. Verfahren gemäss Anspruch 1, worin die Verbindung der Formel I ausgewählt ist aus der Gruppe bestehend aus N-Lauroylaminopropyl-N,N-dimethylglycin, N-Cocoylaminopropyl-N,N-dimethylglycin und Tris-[3-(N-cocoylaminoethyl-N-hydroxyethyl-N-carboxymethyl)amino-2-hydroxypropanol]-phosphat.

6. Verfahren gemäss Anspruch 1, worin die Lösung
   a) zwischen etwa 0,005 und etwa 2,0 Gew.-% einer Verbindung der Formel I,
   b) zwischen 0 und etwa 5 Gew.-% eines vorwiegend nichtionischen Tensids,
   c) zwischen 0 und etwa 5 Gew.-% eines Verdickungsmittels,
   d) zwischen 0 und etwa 1 Gew.-% eines Chelatbildners,
   e) zwischen 0 und etwa 2 Gew.-% eines Puffers,
   f) zwischen 0,5 und etwa 2 Gew.-% eines wasserlöslichen Salzes, das mit dem Augengewebe verträglich ist, und

g) im übrigen Wasser enthält.

**7.** Verfahren gemäss Anspruch 6, worin die Lösung einen Salzgehalt hat, der einem Gehalt von bis zu etwa 0,9 % Natriumchlorid entspricht.

**8.** Verwendung einer Lösung gemäss Anspruch 6 zur Konservierung von Weichkontaktlinsen.

**9.** Verwendung einer Lösung gemäss Anspruch 7 zur Reinigung von Weichkontaktlinsen.

**10.** Verfahren zur Reinigung oder Konservierung einer Weichkontaktlinse, dadurch gekennzeichnet, dass eine solche Kontaktlinse intensiv mit einer Lösung gemäss Anspruch 1 in Kontakt gebracht wird.

**Claims**

**Claims for the following Contracting States : BE, CH, DE, FR, GB, IT, LI, LU, NL, SE**

**1.** An aqueous self-preserving soft contact lens cleaning or preserving solution comprising, as its only preservative agent, an effective surfactant and solution preserving amount of a water-soluble amphoteric surfactant of the formula I

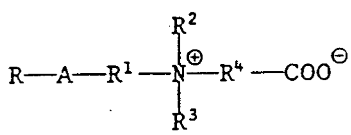

wherein R is alkyl, alkenyl or alkanedienyl of 6 to 18 carbon atoms, each of those radicals being unsubstituted or substituted by halo, hydroxy or amino; A is -O-, -S-, -C(O)O- or -C(O)NR'- where R' is hydrogen or lower alkyl; $R^1$ is alkylene of 2 to 6 carbon atoms which is unsubstituted or substituted by hydroxy; $R^2$ and $R^3$ are independently hydrogen or lower alkyl which is unsubstituted or substituted by carboxy or by one or two hydroxyls, one of which may be esterified with phosphoric or sulfuric acid; and $R^4$ is alkylene of up to 3 carbon atoms which is unsubstituted or substituted by hydroxy; or an ophthalmologically acceptable salt thereof,
as well as an ophthalmologically acceptable water-soluble salt which is compatible with ocular tissue, in such an amount to provide a solution salt content equivalent in tonicity to about 0.5 to about 2 % by weight sodium chloride.

**2.** A solution according to claim 1 wherein R is alkyl or alkenyl of 11 to 17 carbon atoms, A is -O- or -C(O)NH-, the carbonyl group of which is attached to R, $R^1$ is alkylene of 2 or 3 carbon atoms which is unsubstituted or substituted by hydroxy, $R^2$ and $R^3$ are independently lower alkyl which is unsubstituted or monosubstituted by carboxy or mono- or di-substituted by hydroxy, one hydroxy of which may be esterified with phosphoric acid to form the corresponding triorganophosphate, and $R^4$ is alkylene of up to 3 carbon atoms.

**3.** A solution according to claim 1 wherein R is alkyl of 11 to 17 carbon atoms or alkenyl of 17 carbon atoms, A is -C(O)NH-, the carbonyl group of which is attached to R, $R^1$ is alkylene of 2 or 3 carbon atoms, $R^2$ and $R^3$ are independently lower alkyl which is unsubstituted or monosubstituted by hydroxy, which may be esterified with phosphoric acid to form the corresponding triorganophosphate, and $R^4$ is alkylene of up to 3 carbon atoms.

**4.** A solution according to claim 1 wherein R is alkyl or alkenyl of 8 to 12 carbon atoms, A is -C(O)NH-, the carbonyl group of which is attached to R, $R^1$ is alkylene of 2 or 3 carbon atoms, $R^2$ and $R^3$ are independently lower alkyl, and $R^4$ is alkylene of up to 3 carbon atoms.

**5.** A solution according to claim 1 wherein the compound of formula I is selected from the group consisting of N-lauroylaminopropyl-N,N-dimethyl glycine; N-cocoylaminopropyl-N,N-dimethyl glycine and tris-[3-(N-cocoylaminoethyl-N-hydroxyethyl-N-carboxymethyl)amino-2-hydroxy-propanol]phosphate.

**6.** The solution of claim 1 comprising:

a) between about 0.005 and about 2.0 % by weight of a compound of formula I;
b) between 0 and about 5 % by weight of a substantially nonionic surfactant;
c) between 0 and about 5 % by weight of a thickener;
d) between 0 and about 1 % by weight of a chelating agent;
e) between 0 and about 2 % by weight of a buffer;
f) between 0.5 and about 2 % by weight of a water-soluble salt compatible with ocular tissue; and
g) the remainder water.

**7.** The solution of claim 6 having a salt content equivalent to up to about 0.9 % sodium chloride.

**8.** The use of a solution according to claim 6 for preserving soft contact lenses.

**9.** The use of a solution according to claim 7 for cleaning soft contact lenses.

**10.** A method of cleaning or preserving a soft contact lens, comprising intimately contacting said soft contact lens with a solution according to claim 1.

**11.** A process for the manufacture of a solution according to claim 1 by conventionally mixing the ingredients.

**Claims for the following Contracting States : AT, ES**

**1.** A process for the manufacture of an aqueous self-preserving soft contact lens cleaning or preserving solution comprising, as its only preservative agent, an effective surfactant and solution preserving amount of a water-soluble amphoteric surfactant of the formula I

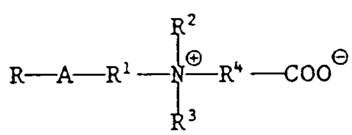

wherein R is alkyl, alkenyl or alkanedienyl of 6 to 18 carbon atoms, each of those radicals being unsubstituted or substituted by halo, hydroxy or amino; A is -O-, -S-, -C(O)O- or -C(O)NR'- where R' is hydrogen or lower alkyl; $R^1$ is alkylene of 2 to 6 carbon atoms which is unsubstituted or substituted by hydroxy; $R^2$ and $R^3$ are independently hydrogen or lower alkyl which is unsubstituted or substituted by carboxy or by one or two hydroxyls, one of which may be esterified with phosphoric or sulfuric acid; and $R^4$ is alkylene of up to 3 carbon atoms which is unsubstituted or substituted by hydroxy; or an ophthalmologically acceptable salt thereof,
as well as an ophthalmologically acceptable water-soluble salt which is compatible with ocular tissue, in such an amount to provide a solution salt content equivalent in tonicity to about 0.5 to about 2 % by weight sodium chloride,
which process comprises conventionally mixing the ingredients.

**2.** A process according to claim 1 wherein R is alkyl or alkenyl of 11 to 17 carbon atoms, A is -O- or -C-(O)NH-, the carbonyl group of which is attached to R, $R^1$ is alkylene of 2 or 3 carbon atoms which is unsubstituted or substituted by hydroxy, $R^2$ and $R^3$ are independently lower alkyl which is unsubstituted or monosubstituted by carboxy or mono- or di-substituted by hydroxy, one hydroxy of which may be esterified with phosphoric acid to form the corresponding triorganophosphate, and $R^4$ is alkylene of up to 3 carbon atoms.

**3.** A process according to claim 1 wherein R is alkyl of 11 to 17 carbon atoms or alkenyl of 17 carbon atoms, A is -C(O)NH-, the carbonyl group of which is attached to R, $R^1$ is alkylene of 2 or 3 carbon atoms, $R^2$ and $R^3$ are independently lower alkyl which is unsubstituted or monosubstituted by hydroxy, which may be esterified with phosphoric acid to form the corresponding triorganophosphate, and $R^4$ is alkylene of up to 3 carbon atoms.

**4.** A process according to claim 1 wherein R is alkyl or alkenyl of 8 to 12 carbon atoms, A is -C(O)NH-,

the carbonyl group of which is attached to R, $R^1$ is alkylene of 2 or 3 carbon atoms, $R^2$ and $R^3$ are independently lower alkyl, and $R^4$ is alkylene of up to 3 carbon atoms.

**5.** A process according to claim 1 wherein the compound of formula I is selected from the group consisting of N-lauroylaminopropyl-N,N-dimethyl glycine; N-cocoylaminopropyl-N,N-dimethyl glycine and tris-[3-(N-cocoylamino-ethyl-N-hydroxyethyl-N-carboxymethyl)amino-2-hydroxypropanol phosphate.

**6.** A process according to claim 1 wherein the solution comprises:
a) between about 0.005 and about 2.0 % by weight of a compound of formula I;
b) between 0 and about 5 % by weight of a substantially nonionic surfactant;
c) between 0 and about 5 % by weight of a thickener;
d) between 0 and about 1 % by weight of a chelating agent;
e) between 0 and about 2 % by weight of a buffer;
f) between 0.5 and about 2 % by weight of a water-soluble salt compatible with ocular tissue; and
g) the remainder water.

**7.** A process according to claim 6 wherein the solution has a salt content equivalent to up to about 0.9 % sodium chloride.

**8.** The use of a solution according to claim 6 for preserving soft contact lenses.

**9.** The use of a solution according to claim 7 for cleaning soft contact lenses.

**10.** A method of cleaning or preserving a soft contact lens, comprising intimately contacting said soft contact lens with a solution according to claim 1.

**Revendications**
**Revendications pour les Etats contractants suivants : BE, CH, DE, FR, GB, GR, IT, LI, LU, NL, SE**

**1.** Solution aqueuse, se conservant d'elle-même, convenant pour le nettoyage ou la conservation des lentilles de contact souples, contenant en tant qu'unique conservateur, en quantité efficace en égard aux propriétés recherc hées d'agent tensio-actif et conservateur, un agent tensio-actif amphotère soluble dans l'eau de formule I

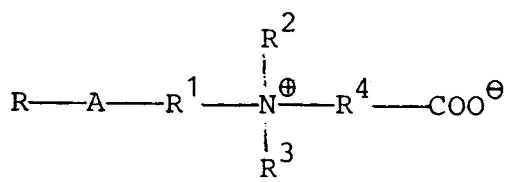

dans laquelle R représente un groupe alkyle, alcényle ou alcane-diényle en C6-C18, chacun de ces groupes pouvant être non substitué ou substitué par des halogènes, des groupes hydroxy ou amino, A représente -O-, -S-, -C(O)O-ou -C(O)NR'- dans lequel R' représente l'hydrogène ou un groupe alkyle inférieur, $R^1$ représente un groupe alkylène en C2-C6 non substitué ou substitué par des groupes hydroxy, $R^2$ et $R^3$ représentent chacun, indépendamment l'un de l'autre, l'hydrogène ou un groupe alkyle inférieur non substitué ou substitué par un groupe carboxy ou par un ou deux groupes hydroxy dont l'un peut être estérifié par l'acide phosphorique ou l'acide sulfurique, et $R^4$ représente un groupe alkylène contenant jusqu'à 3 atomes de carbone, non substitué ou substitué par des groupes hydroxy, ou un sel d'un tel composé acceptable pour les usages ophtalmologiques, avec un sel soluble dans l'eau, acceptable pour les usages ophtalmologiques, toléré par les tissus oculaires, en quantité suffisante pour que la solution ait une teneur en sel correspondant à une tonicité d'environ 0,5 à 2 % en poids de chlorure de sodium.

**2.** Une solution selon revendication 1, pour laquelle R représente un groupe alkyle ou alcényle en C11-C17, A représente -O- ou -C(O)NH- dont le groupe carbonyle est relié à R, $R^1$ représente un groupe alkylène en C2 ou C3 non substitué ou substitué par des groupes hydroxy, $R^2$ et $R^3$ représentent chacun, indépendamment l'un de l'autre, un groupe alkyle inférieur non substitué ou monosubstitué par

un groupe carboxy ou mono- ou disubstitué par des groupes hydroxy dont l'un peut être estérifié par l'acide phosphorique avec formation du triorganophosphate correspondant, et $R^4$ représente un groupe alkylène contenant jusqu'à 3 atomes de carbone.

**3.** Une solution selon revendication 1, pour laquelle R représente un groupe alkyle en C11-C17 ou alcényle contenant jusqu'à 17 atomes de carbone, A représente -C(O)NH- dont le groupe carbonyle est relié à R, $R^1$ représente un groupe alkylène en C2 ou C3, $R^2$ et $R^3$ représentent chacun, indépendamment l'un de l'autre, un groupe alkyle inférieur non substitué ou monosubstitué par un groupe hydroxy, lequel peut être estérifié par l'acide phosphorique avec formation du triorganophosphate correspondant, et $R^4$ représente un groupe alkylène contenant jusqu'à 3 atomes de carbone.

**4.** Une solution selon la revendication 1, pour laquelle R représente un groupe alkyle ou alcényle en C8 à C12, A représente -C(O)NH- dont le groupe carbonyle est relié à R, $R^1$ représente un groupe alkylène en C2 ou C3, $R^2$ et $R^3$ représentent chacun, indépendamment l'un de l'autre, un groupe alkyle inférieur, et $R^4$ un groupe alkylène contenant jusqu'à 3 atomes de carbone.

**5.** Une solution selon revendication 1, dans laquelle le composé de formule I est choisi dans le groupe formé par la N-lauroylaminopropyl-N,N-diméthylglycine, la N-cocoylaminopropyl-N,N-diméthylglycine et le phosphate de tris-[3-(N-cocoylaminoéthyl-N-hydroxyéthyl-N-carboxy-méthyl)-amino-2-hydroxypropanol].

**6.** La solution selon revendication 1 qui contient
a) de 0,005 à 2,0 % en poids environ d'un composé de formule I,
b) de 0 à environ 5 % en poids d'un agent tensio-actif principalement non-ionique,
c) de 0 à environ 5 % en poids d'un agent épaississant,
d) de 0 à environ 1 % en poids d'un agent séquestrant,
e) de 0 à environ 2 % en poids d'un tampon,
f) de 0,5 à environ 2 % en poids d'un sel soluble dans l'eau toléré par les tissus oculaires et
g) de l'eau pour le complément à 100 %.

**7.** La solution selon revendication 6, à une teneur en sel correspondant à une teneur allant jusqu'à environ 0,9 % de chlorure de sodium.

**8.** Utilisation d'une solution selon revendication 6 pour la conservation des lentilles de contact souples.

**9.** Utilisation d'une solution selon revendication 7, pour le nettoyage des lentilles de contact souples.

**10.** Procédé pour nettoyer ou conserver une lentille de contact souple, caractérisé en ce que l'on met la lentille de contact en contact intensif avec une solution selon revendication 1.

**11.** Procédé de préparation d'une solution selon revendication 1 par mélange, de manière connue en soi, des composants.

**Revendications pour les Etats contractants suivants : AT, ES**

**1.** Procédé de préparation d'une solution aqueuse, se conservant d'elle-même, convenant pour le nettoyage ou la conservation des lentilles de contact souples, contenant en tant qu'unique conservateur, en quantité efficace en égard aux propriétés recherchées d'agent tensio-actif et conservateur, un agent tensio-actif amphotère soluble dans l'eau de formule I

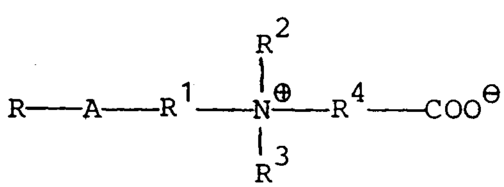

dans laquelle R représente un groupe alkyle, alcényle ou alcane-diényle en C6-C18, chacun de ces

groupes pouvant être non substitué ou substitué par des halogènes, des groupes hydroxy ou amino, A représente -O-, -S-, -C(O)O-ou -C(O)NR'- dans lequel R' représente l'hydrogène ou un groupe alkyle inférieur, $R^1$ représente un groupe alkylène en C2-C6 non substitué ou substitué par des groupes hydroxy, $R^2$ et $R^3$ représentent chacun, indépendamment l'un de l'autre, l'hydrogène ou un groupe alkyle inférieur non substitué ou substitué par un groupe carboxy ou par un ou deux groupes hydroxy dont l'un peut être estérifié par l'acide phosphorique ou l'acide sulfurique, et $R^4$ représente un groupe alkylène non substitué ou substitué par des groupes hydroxy et contenant jusqu'à 3 atomes de carbone, ou un sel d'un tel composé acceptable pour les usages ophtalmologiques, ainsi qu'un sel soluble dans l'eau, acceptable pour les usages ophtalmologiques, toléré par les tissus oculaires, en quantité suffisante pour que la solution ait une teneur en sel correspondant à une tonicité d'environ 0,5 à 2 % en poids de chlorure de sodium, caractérisé en ce que l'on mélange les constituants de manière connue en soi.

**2.** Procédé selon la revendication 1, dans lequel R représente un groupe alkyle ou alcényle en C11-C17, A représente -O- ou -C(O)NH- dont le groupe carbonyle est relié à R, $R^1$ représente un groupe alkylène en C2 ou C3 non substitué ou substitué par des groupes hydroxy, $R^2$ et $R^3$ représentent chacun, indépendamment l'un de l'autre, un groupe alkyle inférieur non substitué ou monosubstitué par un groupe carboxy ou mono- ou di-substitué par des groupes hydroxy, l'un de ces groupes hydroxy pouvant être estérifié par l'acide phosphorique avec formation du triorganophosphate correspondant, et $R^4$ représente un groupe alkylène contenant jusqu'à 3 atomes de carbone.

**3.** Procédé selon la revendication 1 dans lequel R représente un groupe alkyle en C11-C17 ou alcényle contenant jusqu'à 17 atomes de carbone, A représente -C(O)NH- dont le groupe carbonyle est relié à R, $R^1$ représente un groupe alkylène en C2 ou C3, $R^3$ et $R^3$ représentent chacun, indépendamment l'un de l'autre, un groupe alkyle inférieur non substitué ou monosubstitué par un groupe hydroxy, ce dernier pouvant être estérifié par l'acide phosphorique avec formation du triorganophosphate correspondant, et $R^4$ représente un groupe alkylène contenant jusqu'à 3 atomes de carbone.

**4.** Procédé selon la revendication 1, dans lequel R représente un groupe alkyle ou alcényle en ce à C12, A représente -C(O)NH- dont le groupe carbonyle est relié à R, $R^1$ représente un groupe alkylène en C2 ou C3, $R^2$ et $R^3$ représentent chacun, indépendamment l'un de l'autre, un groupe alkyle inférieur, et $R^4$ un groupe alkylène contenant jusqu'à 3 atomes de carbone.

**5.** Procédé selon la revendication 1, dans lequel le composé de formule I est choisi dans le groupe consistant en la N-lauroylaminopropyl-N,N-diméthylglycine, la N-cocoylaminopropyl-N,N-diméthylglycine et le phosphate de tris-[3-(N-cocoylaminoéthyl-N-hydroxyéthyl-N-carboxy-méthyl)-amino-2-hydroxy-propanol].

**6.** Procédé selon la revendication 1, dans lequel la solution contient :
a) d'environ 0,005 à environ 2,0 % en poids d'un composé de formule I,
b) de 0 à environ 5 % en poids d'un agent tensio-actif principalement non-ionique,
c) de 0 à environ 5 % en poids d'un agent épaississant,
d) de 0 à environ 1 % en poids d'un agent séquestrant,
e) de 0 à environ 2 % en poids d'un tampon,
f) de 0,5 à environ 2 % en poids d'un sel soluble dans l'eau toléré par les tissus oculaires et
g) de l'eau pour le complément à 100 %.

**7.** Procédé selon la revendication 6, dans lequel la solution a une teneur en sel correspondant à une teneur en chlorure de sodium allant jusqu'à environ 0,9 %.

**8.** Utilisation d'une solution selon la revendication 6 pour la conservation des lentilles de contact souples.

**9.** Utilisation d'une solution selon revendication 7 pour le nettoyage des lentilles de contact souples.

**10.** Procédé pour nettoyer ou conserver une lentille de contact souple, caractérisé en ce que l'on met la lentille de contact en contact intensif avec une solution selon la revendication 1.